# EUROPÄISCHE PATENTSCHRIFT

(11) **EP 2 313 377 B1**
(45) Veröffentlichungstag und Bekanntmachung des Hinweises auf die Patenterteilung: **06.01.2016**
(21) Anmeldenummer: 09804535.4
(22) Anmeldetag: 23.07.2009
(51) Int. Cl.: C07D 241/08, C07D 471/20

(54) **VERFAHREN ZUR STEREOSELEKTIVEN SYNTHESE BICYCLISCHER HETEROCYCLEN**
METHOD FOR STEREOSELECTIVE SYNTHESIS OF BICYCLIC HETEROCYCLES
PROCÉDÉ DE SYNTHÈSE STÉRÉOSÉLECTIVE D'HÉTÉROCYCLES BICYCLIQUES

(30) Priorität: 08.08.2008 EP 08104993
(43) Veröffentlichungstag der Anmeldung: 27.04.2011
(73) Patentinhaber: BOEHRINGER INGELHEIM INTERNATIONAL GMBH, 55216 Ingelheim (DE)
(72) Erfinder: OSTERMEIER, Markus, 55216 Ingelheim am Rhein (DE); BRAITH, Stefan, 55216 Ingelheim am Rhein (DE); DAEUBLER, Juergen, 55216 Ingelheim am Rhein (DE); HUCHLER, Guenther, 55216 Ingelheim am Rhein (DE); PFRENGLE, Waldemar, 55216 Ingelheim am Rhein (DE)
(74) Vertreter: Simon, Elke Anna Maria
(86) Internationale Anmeldenummer: PCT/EP2009/059512
(87) Internationale Veröffentlichungsnummer: WO 2010/015524

(56) Entgegenhaltungen:
- EP-A- 1 921 070
- WO-A-03/082831
- WO-A-2008/095847

## Beschreibung

Gegenstand der vorliegenden Erfindung ist ein Verfahren zur stereoselektiven Herstellung von Verbindungen der allgemeinen Formel (I) 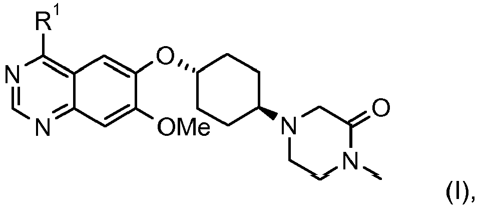 und deren Salze, insbesondere deren physiologisch verträgliche Salze mit anorganischen oder organischen Säuren und Basen, welche wertvolle pharmakologische Eigenschaften aufweisen, insbesondere eine Hemmwirkung auf die durch Tyrosinkinasen vermittelte Signaltransduktion, deren Verwendung zur Behandlung von Krankheiten, insbesondere von Tumorerkrankungen sowie der benignen Prostatahyperplasie (BPH), von Erkrankungen der Lunge und der Atemwege.

### Hintergrund der Erfindung

Chinazolinderivate sind aus dem Stand der Technik als Wirkstoffe beispielsweise zur Behandlung von Tumorerkrankungen als auch von Erkrankungen der Lunge und der Atemwege bekannt. Verfahren zur Herstellung von Chinazolinderivaten werden in WO03082831 sowie in EP1921070 beschrieben.

Es ist die Aufgabe der vorliegenden Erfindung ein stereoselektives Verfahren zur Herstellung der erfindungsgemäßen Chinazolinderivate bereitzustellen.

### Beschreibung der Erfindung

Die vorliegende Erfindung löst die vorstehend genannte Aufgabe über das im Folgenden beschriebene Syntheseverfahren.

Gegenstand der Erfindung ist somit ein Verfahren zur stereoselektiven Herstellung von Verbindungen der allgemeinen Formel **(I),** 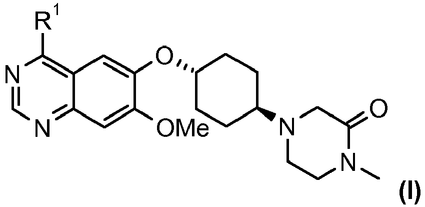 gegebenenfalls in Form ihrer Tautomeren, sowie gegebenenfalls ihrer pharmakologisch unbedenklichen Säureadditionssalze,
worin
**R¹** ein Rest ausgewählt aus der Gruppe bestehend aus 3-Chlor-2-fluor-phenyl-amino-, 3-Chlor-4-fluor-phenylamino-, 2-Fluor-3-methyl-phenylamino-, 2,5-Difluor-3-methyl-phenylamino-, 3-Chlor-2-methyl-phenylamino- und 2-Fluor-5-methyl-phenylamino-, vorzugsweise 3-Chlor-2-fluor-phenyl-amino- oder 3-Chlor-4-fluor-phenylamino-, besonders bevorzugt 3-Chlor-2-fluor-phenyl-amino-,
bedeutet,
wobei das Verfahren die Reaktionsschritte **(1a)** bis **(1d)** umfasst, worin
**(1a)** die Umsetzung einer Verbindung der Formel **(II)** 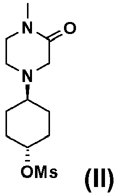 mit einer Verbindung der der Formel **(III)** 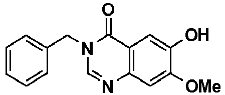 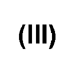 zu einer Verbindung der Formel **(IV)** 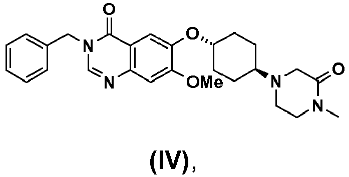
**(1b)** die Abspaltung des Benzylrestes der Verbindung der Formel **(IV)** in Anwesenheit eines Katalysators zu einer Verbindung der Formel **(V)** 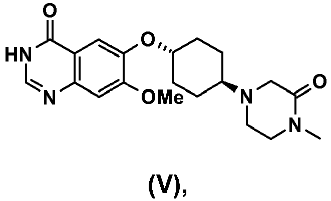
**(1c)** die Umsetzung der Verbindung der Formel **(V)** mit einem Chlorierungsmittel zu dem Hydrochlorid einer Verbindung der Formel **(VI)** 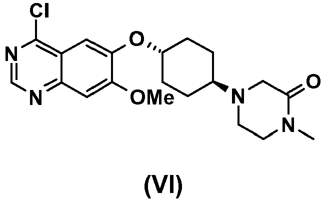 und
**(1d)** die Umsetzung der Verbindung der Formel **(VI)** mit einer der Verbindungen **(i)** bis **(vi)** zu einer Verbindung der Formel **(I),**
   wobei
   **(i)** 3-Chlor-2-fluor-anilin,
   **(ii)** 3-Chlor-4-fluor-anilin,
   **(ii)** 2-Fluor-3-methyl-anilin,
   **(iii)** 2,5-Difluor-3-methyl-anilin,
   **(iv)** 3-Chlor-2-methyl-anilin, und
   **(vi)** 2-Fluor-5-methyl-anilin,
   vorzugsweise 3-Chlor-2-fluor-anilin oder 3-Chlor-4-fluor-anilin, besonders bevorzugt 3-Chlor-2-fluor-anilin,
   bedeutet,
   wobei die Schritte **(1a)** bis **(1d)** in der genannten Reihenfolge nacheinander erfolgen, oder
   wobei das Verfahren die Reaktionsschritte **(2a), (1c)** und **(1d)** umfasst, worin
**(2a)** die Umsetzung einer Verbindung der Formel **(II)** 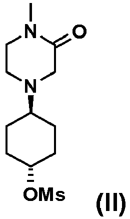 mit einer Verbindung der der Formel **(VII)** 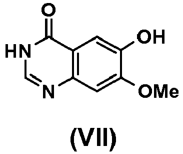 zu einer Verbindung der Formel **(V)** 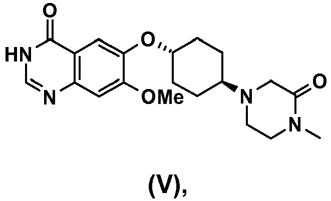
bedeutet,
wobei die Schritte in der genannten Reihenfolge nacheinander erfolgen.

Bevorzugt ist ein Verfahren zur stereoselektiven Herstellung von Verbindungen der allgemeinen Formel **(I),** wobei das Verfahren aus den Verfahrensschritten **(1a)** bis **(1 d)** besteht.

Ferner bevorzugt ist ein Verfahren zur stereoselektiven Herstellung von Verbindungen der allgemeinen Formel **(I),** wobei das Verfahren aus den Verfahrensschritten **(2a), (1c)** und **(1d)** besteht.

Ferner bevorzugt ist ein Verfahren zur stereoselektiven Herstellung von Verbindungen der allgemeinen Formel **(IV),** dadurch gekennzeichnet dass das Verfahren aus dem Verfahrensschritt **(1a)** besteht.

Ferner bevorzugt ist ein Verfahren zur stereoselektiven Herstellung von Verbindungen der allgemeinen Formel **(V),** dadurch gekennzeichnet dass das Verfahren aus dem Verfahrensschritt **(1b)** besteht.

Ferner bevorzugt ist ein Verfahren zur stereoselektiven Herstellung von Verbindungen der allgemeinen Formel **(VI),** dadurch gekennzeichnet dass das Verfahren aus dem Verfahrensschritt **(1c)** besteht.

Ferner bevorzugt ist ein Verfahren zur stereoselektiven Herstellung von Verbindungen der allgemeinen Formel **(I),** dadurch gekennzeichnet dass das Verfahren aus dem Verfahrensschritt **(1d)** besteht.

Ferner bevorzugt ist ein Verfahren zur stereoselektiven Herstellung von Verbindungen der allgemeinen Formel **(IV),** dadurch gekennzeichnet dass das Verfahren aus dem Verfahrensschritt **(2a)** besteht.

Insbesondere bevorzugt ist ein Verfahren, worin in Verfahrensschritt **(1b)** ein Palladium/Kohle Katalysator eingesetzt wird.

Weiterhin Insbesondere bevorzugt ist ein Verfahren, worin ein Chlorierungsmittel ausgewählt aus der Gruppe bestehend aus Oxalylchlorid, Thionylchlorid, Phosphoroxychlorid, Phosphorpentachlorid, einer N-Chlorsuccinimid/ Triphenylphosphan - Kombination, einer Tetrachlorkohlenstoff/Triphenylphosphan-Kombination, Dichlortriphenylphosphoran und P,P-Dichlor-phenylphosphinoxid eingesetzt wird.

Ein weiterer Gegenstand der Erfindung ist die Verbindung der Formel **(II).**

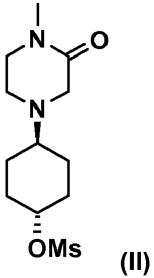

Ein weiterer Gegenstand der Erfindung ist die Verbindung der Formel **(VIII).**

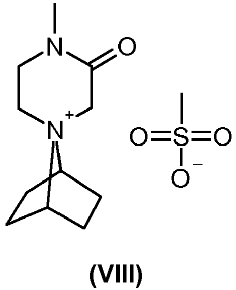

Die erfindungsgemäßen Verbindungen können in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, anorganischen Säuren, beispielsweise Phosphorsäure oder Schwefelsäure oder organischen Säuren, wie beispielsweise Oxal-, Fumar-, Diglycol-, Toluolsulfon-, Benzoe-, Bernstein-, oder Methansulfonsäure, vorliegen.

Verfahrensschritt (1a) wird vorzugsweise in einem Lösungsmittel ausgewählt aus der Gruppe bestehend aus Dimethylformamid (DMF), N-Methyl-2-pyrrolidon (NMP), N-Ethyl-2-pyrrolidon (NEP) und Dimethylsulfoxid (DMSO), bevorzugt in NMP durchgeführt.

Verfahrensschritt (1a) wird vorzugsweise in einem Temperaturbereich von 70 C° bis 150 C°, bevorzugt von 100 C° bis 145 C°, insbesondere bevorzugt bei einer Temperatur von 140 C°, durchgeführt.

In Verfahrensschritt (1a) werden vorzugsweise Basen ausgewählt aus der Gruppe bestehend aus Na₂CO₃, K₂CO₃, Cs₂CO₃, bevorzugt Na₂CO₃, eingesetzt.

Verfahrensschritt (2a) wird vorzugsweise in einem Lösungsmittel ausgewählt aus der Gruppe bestehend aus DMF, NMP, NEP und DMSO, bevorzugt in NMP durchgeführt.

Verfahrensschritt (2a) wird vorzugsweise in einem Temperaturbereich von 70 C° bis 150 C°, bevorzugt von 100 C° bis 140 C°, insbesondere bevorzugt bei einer Temperatur von 130 C°, durchgeführt.

Verfahrensschritt (1 b) wird vorzugsweise in einem Lösungsmittel ausgewählt aus der Gruppe bestehend aus H₂O, HOAc, EtOH, n-PrOH, i-PrOH, Amylalkohol und NMP, bevorzugt in H₂O/HOAc durchgeführt.

Verfahrensschritt (1 b) wird vorzugsweise in einem Temperaturbereich von 0 C° bis 140 C°, bevorzugt von 60 C° bis 100 C°, insbesondere bevorzugt bei einer Temperatur von 80 C°, durchgeführt.

In Verfahrensschritt (1 b) werden vorzugsweise Katalysatoren ausgewählt aus der Gruppe bestehend aus Pd/C, Pd(OH)₂ bevorzugt Pd/C, eingesetzt.

Verfahrensschritt (1 c) wird vorzugsweise in einem Lösungsmittel ausgewählt aus der Gruppe bestehend aus Dioxan, Acetonitril, Tetrahydrofuran (THF) und Diethylenglycoldimethylether, bevorzugt in Dioxan/Acetonitril durchgeführt. Verfahrensschritt (1 c) wird vorzugsweise in einem Temperaturbereich von 20 C° bis 140 C°, bevorzugt von 70 C°.bis 130 C°, insbesondere bevorzugt bei einer Temperatur von 120 C°, durchgeführt.

Verfahrensschritt (1d) wird vorzugsweise in einem Lösungsmittel ausgewählt aus der Gruppe bestehend aus H₂O, HCl_{wässrig}, NMP und Acetonitril, bevorzugt in HCl_{wässrig} durchgeführt.

In Verfahrensschritt (1d) werden vorzugsweise Hilfssäuren ausgewählt aus der Gruppe bestehend aus HCl, H₂SO₄, H₃PO₄, MsOH und TsOH, bevorzugt HCl, verwendet.

Verfahrensschritt (1d) wird vorzugsweise in einem Temperaturbereich von 5.C° bis 100 C°, bevorzugt von 10 C°.bis 40 C°, insbesondere bevorzugt bei einer Temperatur von 20 C°, durchgeführt.

Schema 1 bis 3 illustrieren die erfindungsgemäße Synthese.

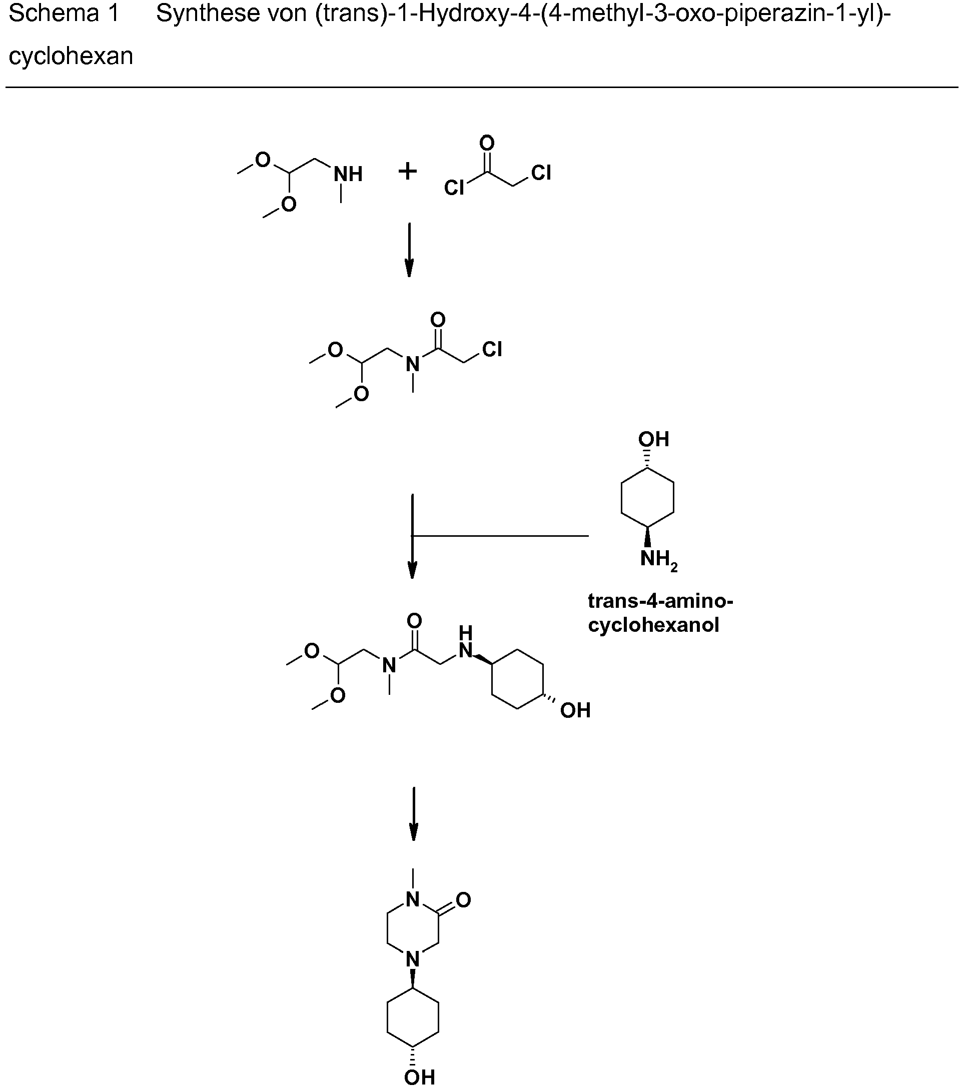

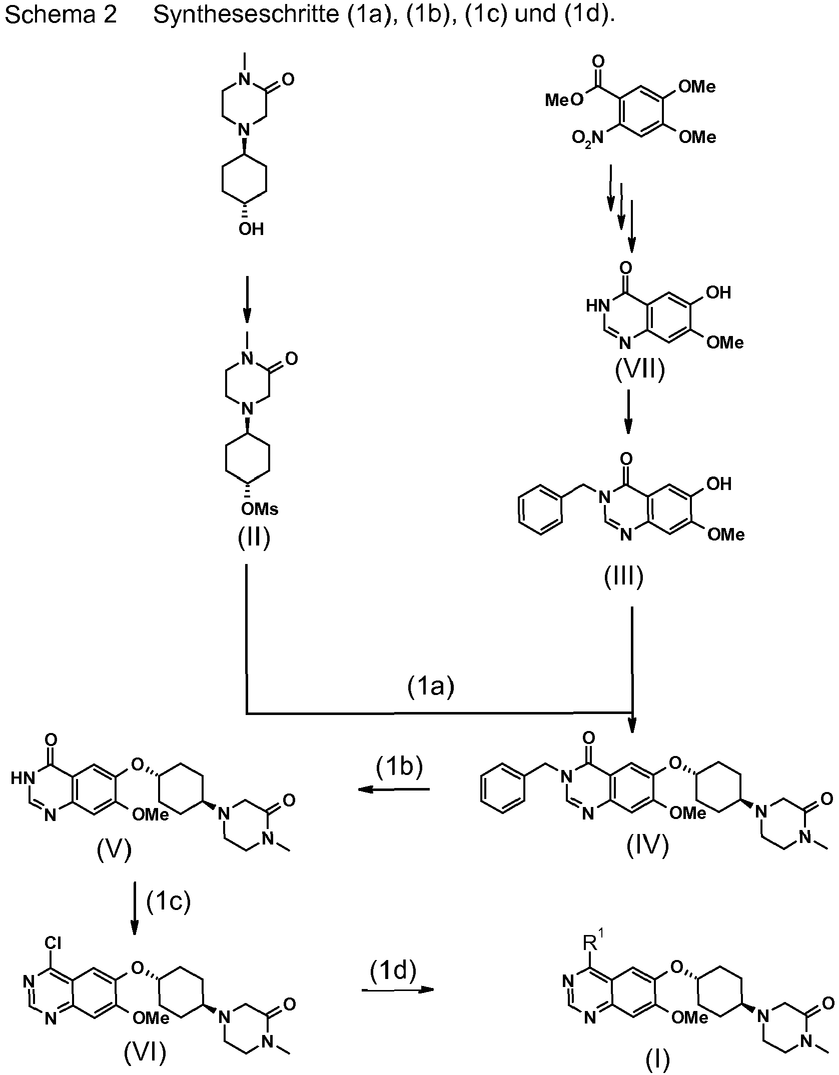

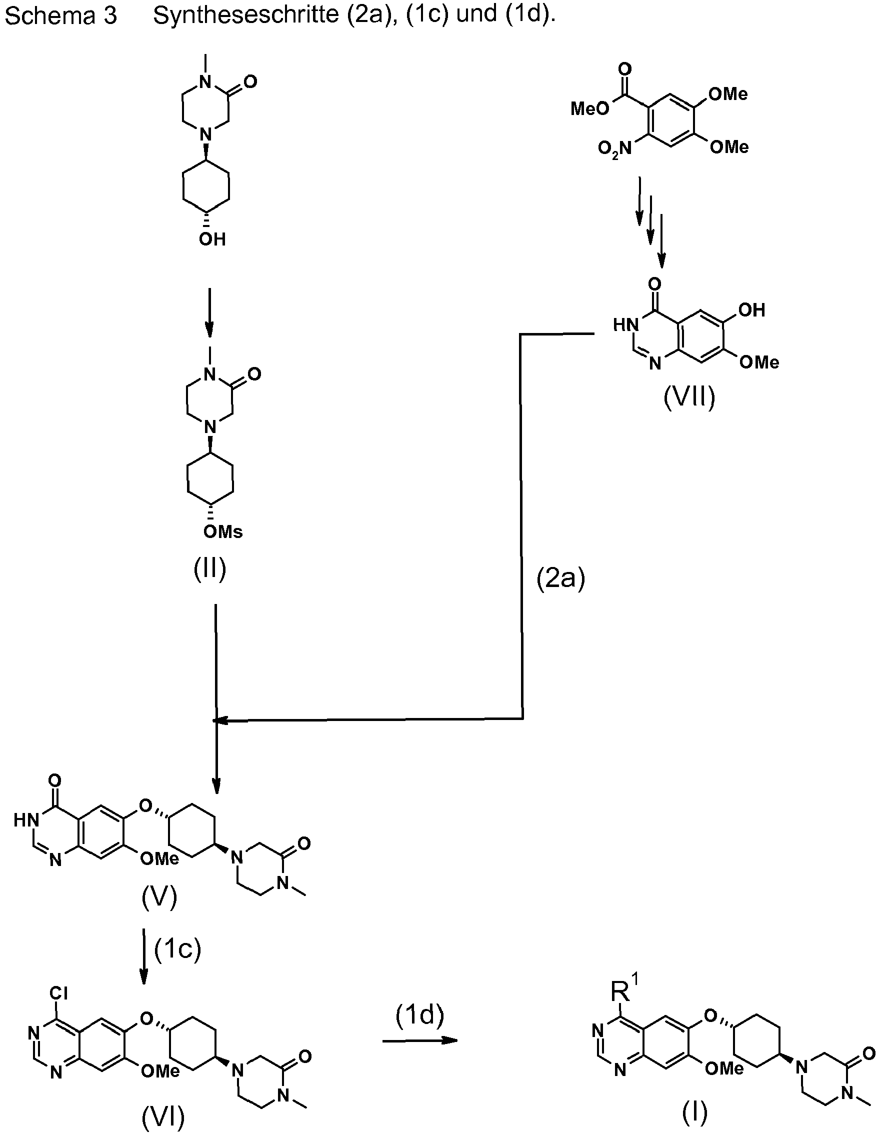

Das nachfolgende Beispiel dient der Illustration des exemplarisch durchgeführten Verfahrens zur Herstellung der Verbindung der Formel (I). Dieses Beispiel ist als Erläuterung der Erfindung zu verstehen, ohne selbiges auf deren Gegenstand zu beschränken.

### Herstellung der Verbindungen gemäß Schema 1

### 2-Chloro-N-(2,2-dimethoxy-ethyl)-N-methyl-acetamid

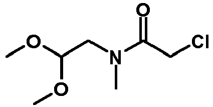

Zu einer Mischung aus 300 ml (Methylamino)-acetaldehyddimethylacetal, 1200 ml 2-Methyltetrahydrofuran und 1200 ml gesättigter Kaliumcarbonatlösung werden bei 2°C 195 ml Chloracetylchlorid in 200 ml 2-Methyltetrahydrofuran innerhalb einer Stunde zugetropft. Nach 40 min werden 1450 ml Wasser zugegeben und die Phasen getrennt. Die wässrige Phase wird mit 600 ml 2-Methyltetrahydrofuran extrahiert. Die vereinigten organischen Phasen werden über Natriumsulfat getrocknet und eingedampft. Es verbleiben 451g Produkt.
Massenspektrum (ESI⁺): m/z = 196 [M+H]⁺

### (trans)-1-Hydroxy-4-{[N-(2,2-dimethoxy-ethyl)-N-methyl-amino]-carbonylmethylamino}-cyclohexan

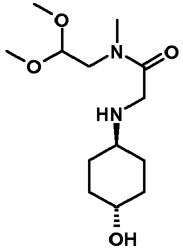

Zu einer Suspension aus 35.3 g trans-4-Aminocyclohexanol und 53 g Kaliumcarbonat in 150 ml n-Butylacetat werden bei 90-100°C innerhalb von 2.5 h 50g 2-Chloro-N-(2,2-dimethoxy-ethyl)-N-methyl-acetamid in 100 ml n-Butylacetat getropft. Nach 45 min wird die Suspension bei 65°C filtriert und mit 70 ml 65°C warmen n-Butylacetat gewaschen. Vom Filtrat werden 100 ml Lösungsmittel abdestilliert und die eingeengte Lösung wird bei 40°C mit Produkt angeimpft. Nach 16 h bei Raumtemperatur wird der Niederschlag abfiltriert und mit 70 ml Tertbutylmethylether gewaschen. Nach Trocknen erhält man 50 g Produkt.
Massenspektrum (ESI⁺): m/z = 275 [M+H]⁺

### (trans)-1-Hydroxy-4-(4-methyl-3-oxo-piperazin-1-yl)-cyclohexan

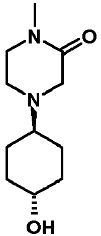

Zu einer Lösung aus 822 g (trans)-1-Hydroxy-4-{[N-(2,2-dimethoxy-ethyl)-N-methyl-amino]-carbonylmethylamino}-cyclohexan in 4500 ml Methanol und 450 ml Wasser werden 82 g Platin auf Kohle (5%) gegeben. Nach Zugabe von 499 ml konzentrierter Salzsäure wird sofort begonnen mit Wasserstoff zu hydrieren. Während des Hydriervorgangs wird auf 50°C erwärmt. Nach 3 h wird filtriert.

Dieser Ansatz wird in gleicher Größe wiederholt.

Die beiden Filtrate der beiden Ansätze werden vereinigt und auf 7100 ml 30%ige Kaliumcarbonatlösung gegossen. Bei 50°C wird mit 15 L tert-Amylalkohol extrahiert. Die wässrige Phase wird noch zweimal mit je 7500 ml tert-Amylalkohol extrahiert. Von den vereinigten organischen Phasen werden 28 L Lösungsmittel abdestilliert. Es werden 6 L n-Butylacetat zugefügt und 8 L Lösungsmittel abdestilliert. Es werden 15 L n-Butylacetat und 1 kg Celite zugefügt und auf 120°C erhitzt. Der Feststoff wird abfiltriert und mit 5000 ml heißem n-Butylacetat gewaschen. Das Filtrat wird auf -5°C abgekühlt. Nach 3 h wird der Niederschlag abfiltriert und mit 5000 ml Tertbutylmethylether gewaschen. Nach Trocknen erhält man 1025 g Produkt.
Massenspektrum (ESI⁺): m/z = 213 [M+H]⁺

### Herstellung der Verbindungen gemäß Schema 2 und 3

### (trans)-1-Methansulfonyloxy-4-(4-methyl-3-oxo-piperazin-1-yl)-cyclohexan (II)

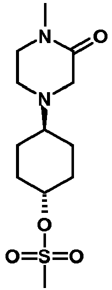

Zu 150 g (trans)-1-Hydroxy-4-(4-methyl-3-oxo-piperazin-1-yl)-cyclohexan und 162 ml Triethylamin in 2300 ml THF werden 109 g Mesylchlorid in 250 ml THF so zugetropft, dass die Temperatur 35°C nicht übersteigt. Nach 25 min wird die Suspension filtriert und mit 300 ml THF nachgewaschen. Das Filtrat wird bei 40°C im Vakkum eingeengt bis auf 520 g Gesamtmasse. Zu der Suspension werden 230 ml Ethylacetat gegeben. Nach 15 min wird filtriert und der Niederschlag mit 230 ml Ethylacetat und 150ml Methyl-tert-butylether gewaschen. Nach Trocknen erhält man 153.1 g Produkt. Massenspektrum (ESI⁺): m/z = 291 [M+H]⁺

### 3-Benzyl-3,4-dihydro-4-oxo-6-[trans-4-(4-methyl-3-oxo-piperazin-1-yl)-cyclohexyloxy]-7-methoxy-chinazolin (IV)

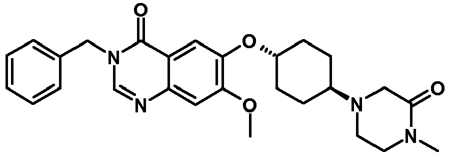

Zu 20 g 3-Benzyl-3,4-dihydro-4-oxo-6-hydroxy-7-methoxy-chinazolin **(III)** und 16.52 g Natriumcarbonat in 160 ml N-Methyl-2-pyrrolidinon werden bei 130°C schrittweise sechsmal je 4.11 g (gesamt: 24.68 g) (trans)-1-Methansulfonyloxy-4-(4-methyl-3-oxo-piperazin-1-yl)-cyclohexan **(II)** innerhalb von 3.5 h zugegeben. Nach 19.5 h wird das Reaktionsgemisch langsam in 400 ml 80°C warmes Wasser eingegossen. Nach 70 min wird die Suspension bei Raumtemperatur abfiltriert und der Niederschlag mit Wasser gewaschen. Das feuchte Rohprodukt wird in 500 ml Ethanol und 275 ml Wasser bei Rückfluss gelöst. Nach Abkühlen auf 5°C und Rühren wird der Niederschlag abgesaugt und getrocknet. Man erhält 26.8 g Produkt.
Massenspektrum (ESI⁺): m/z = 477 [M+H]⁺
oder
Zu 193 g (trans)-1-Hydroxy-4-(4-methyl-3-oxo-piperazin-1-yl)-cyclohexan und 168 ml Triethylamin in 1710 ml THF werden bei 40°C 88 ml Mesylchlorid zugegeben. Die Suspension wird so filtriert, dass das Filtrat direkt in 640 ml N-Methyl-2-pyrrolidinon einfliest. Nach Nachwaschen mit 1280 ml warmen THF wird das Filtrat bei 60°C im Vakuum eingeengt. Der ölige Rückstand wird portionsweise zu 214 g 3-Benzyl-3,4-dihydro-4-oxo-6-hydroxy-7-methoxy-chinazolin **(III)** und 177 g Natriumcarbonat in 640 ml N-Methyl-2-pyrrolidinon bei 130°C gegeben. Es werden 340 ml N-Methyl-2-pyrrolidinon zugegeben. Nach 16 h werden bei 95°C 2140 ml Wasser zugegeben. Nach 80 min wird der Niederschlag abfiltriert und mit 3000 ml Wasser gewaschen. Nach Trocknen erhält man 346 g Produkt.
Massenspektrum (ESI⁺): m/z = 477 [M+H]⁺

### Spiro[7-azoniabicyclo[2.2.1]heptan-7,4'-[1'-methyl-2'-oxo-4'-piperazinium]]methansulfonat (VIII)

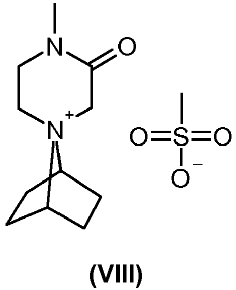

Die Verbindung der Formel **(VIII)** entsteht als Zwischenprodukt des Syntheseschritts (1a). Sie lässt sich ebenfalls herstellen, indem 350mg (trans)-1-Methansulfonyloxy-4-(4-methyl-3-oxo-piperazin-1-yl)-cyclohexan **(II)** für 15.5 h im Vakuumtrockenofen bei 110 °C aufbewahrt werden. 350 mg neues Produkt der Verbindung der Formel **(VIII)** entstehen.
Massenspektrum (ESI⁺): m/z = 195 [M]⁺

### 3,4-Dihydro-4-oxo-6-[trans-4-(4-methyl-3-oxo-piperazin-1-yl)-cyclohexyloxy]-7-methoxy-chinazolin hydrochlorid [(V) HCl]

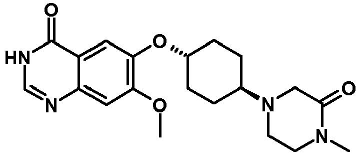

355g 3-Benzyl-3,4-dihydro-4-oxo-6-[trans-4-(4-methyl-3-oxo-piperazin-1-yl)-cyclohexyloxy]-7-methoxy-chinazolin werden in einer Lösung aus 1775 ml Wasser und 1065 ml Eisessig mit 35.5 g Palladium auf Kohle (10% Pd) 23 Stunden lang bei 80°C hydriert. Der Katalysator wird abfiltriert und mit 500 ml Wasser gewaschen. 950 ml 50%ige Natronlauge werden zugegeben. Zur Suspension werden 3000 ml tert-Amylalkohol zugegeben und die Phasen getrennt. Die wässrige Phase wird mit 3000 ml tert-Amylalkohol extrahiert. Die vereinigten organischen Phasen werden eingedampft und der Rückstand wird in 3000 ml Ethanol bei 78°C gelöst. 76 ml von HCl in Ethanol (10 mol/L) werden zugegeben. Nach Animpfen mit Produkthydrochlorid und Kühlen auf -3°C wird der Niederschlag abgesaugt und mit 200 ml Ethanol und 800 ml Methyl-tert-butylether gewaschen. Nach Trocknen erhält man 266g Produkt als Hydrochlorid.
Massenspektrum (ESI+): m/z = 387 [M+H]⁺
oder

### 3,4-Dihydro-4-oxo-6-[trans-4-(4-methyl-3-oxo-piperazin-1-yl)-cyclohexyloxy]-7-methoxy-chinazolin (V)

Zu 2 g 3,4-Dihydro-4-oxo-6-hydroxy-7-methoxy-chinazolin **(VII)** in 16 ml N-Methyl-2-pyrrolidinon werden bei 130°C 2.4 g Natriumcarbonat gegeben. Anschließend werden bei 130°C schrittweise sechsmal je 600 mg (gesamt: 2.4 g) (trans)-1-Methansulfonyloxy-4-(4-methyl-3-oxo-piperazin-1-yl)-cyclohexan **(II)** innerhalb von 4 h zugegeben und der Ansatz für 20 h bei 130°C gehalten. Nach 10 Tagen werden 25 ml Wasser zugegeben und der Ansatz wird mit 25 ml Isopropylacetat, 50ml Dichlormethan und 25ml Dichlormethan extrahiert. Die organischen Phasen werden eingedampft und der Rückstand wird mit präparativer HPLC aufgetrennt. Man erhält 500 mg des Produkts.
Massenspektrum (ESI+): m/z = 387 [M+H]⁺

### 4-Chloro-6-[trans-4-(4-methyl-3-oxo-piperazin-1-yl)-cyclohexyloxy]-7-methoxy-chinazolin hydrochlorid [(VI)HCl]

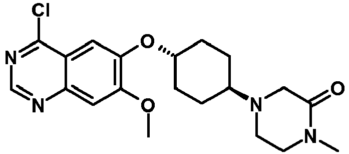

210g 3,4-Dihydro-4-oxo-6-[trans-4-(4-methyl-3-oxo-piperazin-1-yl)-cyclohexyloxy]-7-methoxy-chinazolin hydrochlorid **[(V) HCl]** in 1680 ml Acetonitril werden zum Rückfluss erhitzt und 840 ml Lösungsmittel abdestilliert (Entfernung von Wasserspuren). Es werden 221.4 g Triphenylphosphin in 840 ml Dioxan zugegeben. Es werden 119.4 g N-Chlorsuccinimid in 600 ml Acetonitril zugegeben. Es werden 260 ml Acetonitril zugegeben. Der Ansatz wird 45 min bei 80°C gerührt und dann auf 30°C abgekühlt. Es werden 8.94 ml Wasser und 420 ml Dioxan zugesetzt. Der Niederschlag wird unter Schutzgas abfiltriert und mit 1200 ml THF gewaschen. Das Produkt fällt als Hydrochlorid an und wird ohne Trocknung weiterverarbeitet. Ein kleiner Anteil wurde getrocknet und darauf bezogen kann die Ausbeute mit ca. 210g kalkuliert werden.
Massenspektrum (ESI+): m/z = 405 [M+H]⁺

### 4-[(3-Chlor-2-fluor-phenyl)amino]-6-[trans-4-(4-methyl-3-oxo-piperazin-1-yl)-cyclohexyloxy]-7-methoxy-chinazolin [gemäß allgemeiner Formel (I)]

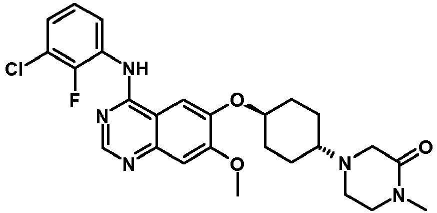

400 g rohes 4-Chloro-6-[trans-4-(4-methyl-3-oxo-piperazin-1-yl)-cyclohexyloxy]-7-methoxy-chinazolin **(VI)** (direkt aus vorheriger Synthese; enthält noch THF - entspricht ca. 210 g 4-Chloro-6-[trans-4-(4-methyl-3-oxo-piperazin-1-yl)-cyclohexyloxy]-7-methoxy-chinazolin hydrochlorid) werden zu 86.7 g 3-Chlor-2-Fluoranilin in 2200 ml 2N Salzsäure bei 20°C gegeben. Nach 1h werden 2200 ml Toluol zugegeben und der Ansatz für 1 h auf 65°C erwärmt. Der Ansatz wird für 19 h bei Raumtemperatur gerührt und dann für 1h bei 6°C. Der Niederschlag wird abgesaugt und mit 400 ml 2N Salzsäure und 400 ml Toluol gewaschen. Nach Trocknen erhält man das Rohprodukt als Hydrochlorid bzw. Dihydrochlorid. Dieses wird in 1900 ml Wasser und 1900 ml Ethanol gelöst. Es werden 920 ml 1 N NaOH zugegeben und 1000 ml Lösungsmittel abdestilliert. Es wird mit Produkt angeimpft und 740 ml Lösungsmittel abdestilliert. Nach Abkühlen auf 7°C wird der Niederschlag mit 1000 ml Wasser gewaschen und getrocknet. Der Niederschlag wird in 7400 ml Ethanol bei 78°C gelöst. 50 g Aktivkohle werden zugegeben und die Lösung wird filtriert und mit 900 ml heißem Ethanol gewaschen. Von der Lösung werden 5300 ml abdestilliert. Es wird mit Produkt angeimpft und 1000 ml Lösungsmittel abdestilliert. Nach Rühren für 66 h bei Raumtemperatur wird der Niederschlag abfiltriert und mit 500 ml Ethanol gewaschen. Nach Trocknen erhält man 191 g Produkt.
Massenspektrum (ESI⁺): m/z = 514 [M+H]⁺

Analog zu vorstehend beschriebener Vorgehensweise werden Verbindungen der allgemeinen Formel (I) hergestellt.

## Patentansprüche

1. Verfahren zur stereoselektiven Herstellung von Verbindungen der allgemeinen Formel **(I)** 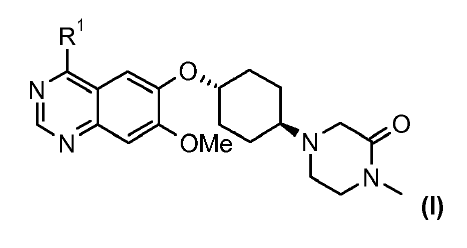 gegebenenfalls in Form ihrer Tautomeren, sowie gegebenenfalls ihrer pharmakologisch unbedenklichen Säureadditionssalze,
worin
**R¹** ein Rest ausgewählt aus der Gruppe bestehend aus 3-Chlor-2-fluorphenyl-amino-, 3-Chlor-4-fluor-phenylamino-, 2-Fluor-3-methyl-phenylamino-, 2,5-Difluor-3-methyl-phenylamino-, 3-Chlor-2-methyl-phenylamino- und 2-Fluor-5-methyl-phenylamino-,
bedeutet,
**dadurch gekennzeichnet, dass** das Verfahren die Reaktionsschritte **(1a)** bis **(1d)** umfasst, wobei
**(1a)** die Umsetzung einer Verbindung der Formel **(II)** 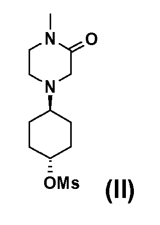 mit einer Verbindung der der Formel **(III)** 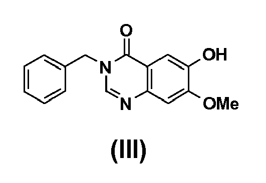 zu einer Verbindung der Formel **(IV)** 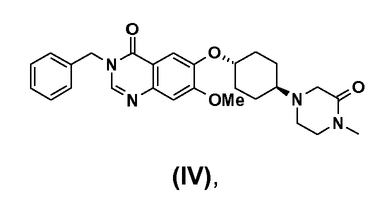
**(1b)** die Abspaltung des Benzylrestes der Verbindung der Formel **(IV)** in Anwesenheit eines Katalysators zu einer Verbindung der Formel **(V)** 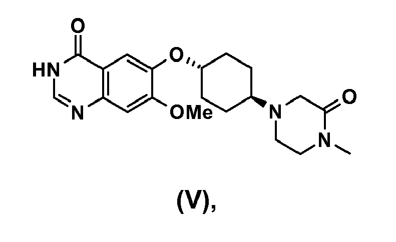
**(1c)** die Umsetzung der Verbindung der Formel **(V)** mit einem Chlorierungsmittel zu dem Hydrochlorid einer Verbindung der Formel **(VI)** 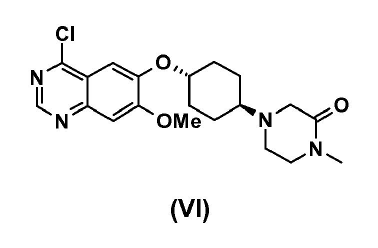 und
**(1d)** die Umsetzung der Verbindung der Formel **(VI)** mit einer der Verbindungen **(i)** bis **(vi)** zu einer Verbindung der Formel **(I),**
wobei
**(i)** 3-Chlor-2-fluor-anilin,
**(ii)** 3-Chlor-4-fluor-anilin,
**(ii)** 2-Fluor-3-methyl-anilin,
**(iii)** 2,5-Difluor-3-methyl-anilin,
**(iv)** 3-Chlor-2-methyl-anilin, und
**(vi)** 2-Fluor-5-methyl-anilin,
bedeutet,
wobei die Schritte **(1a)** bis **(1d)** in der genannten Reihenfolge nacheinander erfolgen,
oder
**dadurch gekennzeichnet, dass** das Verfahren die Reaktionsschritte **(2a), (1c)** und **(1d)** umfasst, wobei
**(2a)** die Umsetzung einer Verbindung der Formel **(II)** 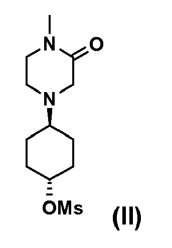 mit einer Verbindung der der Formel **(VII)** 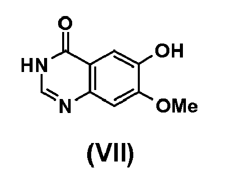 zu einer Verbindung der Formel **(V)** 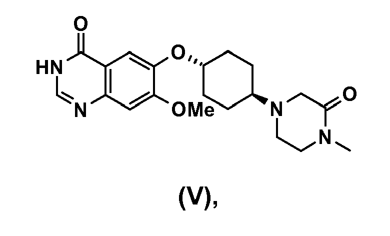 bedeutet,
wobei die Schritte in der genannten Reihenfolge nacheinander erfolgen.

2. Verfahren gemäß Anspruch 1 zur stereoselektiven Herstellung von Verbindungen der allgemeinen Formel **(I), dadurch gekennzeichnet, dass** das Verfahren aus den Verfahrensschritten **(1a)** bis **(1d)** besteht.

3. Verfahren gemäß Anspruch 1 zur stereoselektiven Herstellung von Verbindungen der allgemeinen Formel **(I), dadurch gekennzeichnet, dass** das aus den Verfahrensschritten **(2a), (1c)** und **(1d)** besteht.

4. Verfahren gemäß Anspruch 1 zur stereoselektiven Herstellung von Verbindungen der allgemeinen Formel **(IV), dadurch gekennzeichnet dass** das Verfahren aus dem Verfahrensschritt **(1a)** besteht.

5. Verfahren gemäß Anspruch 1 zur stereoselektiven Herstellung von Verbindungen der allgemeinen Formel **(V), dadurch gekennzeichnet dass** das Verfahren aus dem Verfahrensschritt **(1b)** besteht.

6. Verfahren gemäß Anspruch 1 zur stereoselektiven Herstellung von Verbindungen der allgemeinen Formel **(VI), dadurch gekennzeichnet dass** das Verfahren aus dem Verfahrensschritt **(1c)** besteht.

7. Verfahren gemäß Anspruch 1 zur stereoselektiven Herstellung von Verbindungen der allgemeinen Formel **(I), dadurch gekennzeichnet dass** das Verfahren aus dem Verfahrensschritt **(1d)** besteht.

8. Verfahren gemäß Anspruch 1 zur stereoselektiven Herstellung von Verbindungen der allgemeinen Formel **(IV), dadurch gekennzeichnet dass** das Verfahren aus dem Verfahrensschritt **(2a)** besteht.

9. Verbindung der Formel **(II)**

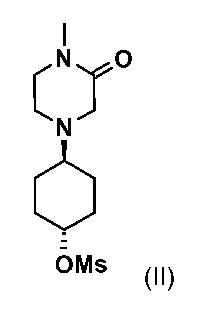

10. Verbindung der Formel **(VIII)**

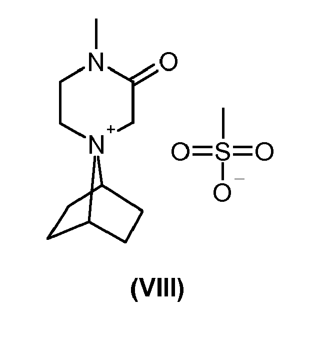

## Claims

1. Process for the stereoselective preparation of compounds of general formula **(I)** 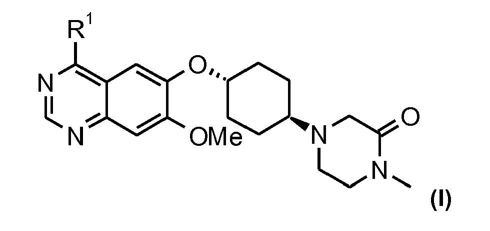 optionally in the form of the tautomers thereof, and optionally the pharmacologically acceptable acid addition salts thereof,
wherein
**R¹** denotes a group selected from among 3-chloro-2-fluoro-phenyl-amino, 3-chloro-4-fluoro-phenylamino, 2-fluoro-3-methyl-phenylamino, 2,5-difluoro-3-methyl-phenylamino, 3-chloro-2-methyl-phenylamino and 2-fluoro-5-methyl-phenylamino,
**characterised in that** the process comprises reaction steps **(1 a)** to **(1 d),** wherein
**(1a)** is the reaction of a compound of formula **(II)** 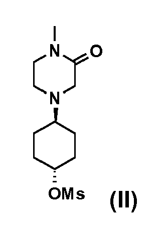 with a compound of formula **(III)** 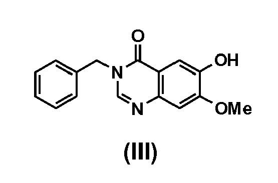 to form a compound of formula **(IV)** 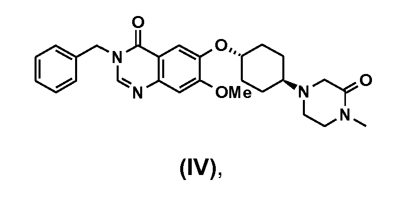
**(1b)** is the cleaving of the benzyl group of the compound of formula **(IV)** in the presence of a catalyst to form a compound of formula **(V)** 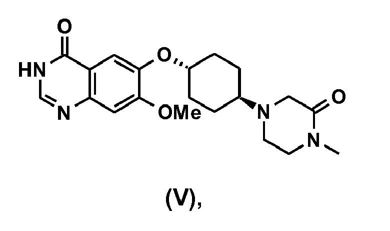
**(1c)** is the reaction of the compound of formula **(V)** with a chlorinating agent to form the hydrochloride of a compound of formula **(VI)** 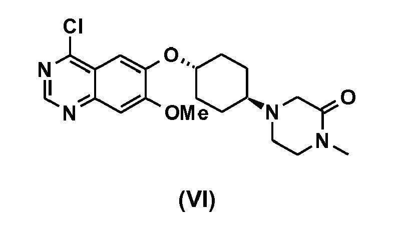 and
**(1d)** is the reaction of the compound of formula **(VI)** with one of the compounds (i) to (vi) to form a compound of formula **(I),**
wherein
**(i)** is 3-chloro-2-fluoro-aniline,
**(ii)** is 3-chloro-4-fluoro-aniline,
**(ii)** is 2-fluoro-3-methyl-aniline,
**(iii)** is 2,5-difluoro-3-methyl-aniline,
**(iv)** is 3-chloro-2-methyl-aniline, and
**(vi)** is 2-fluoro-5-methyl-aniline,
while steps **(1a)** to **(1d)** take place successively in the order specified, or
**characterised in that** the process comprises reaction steps **(2a), (1c)** and **(1d),** wherein
**(2a)** is the reaction of a compound of formula **(II)** 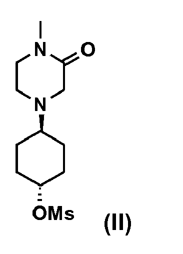 with a compound of formula **(VII)** 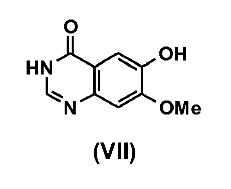 to form a compound of formula **(V)** 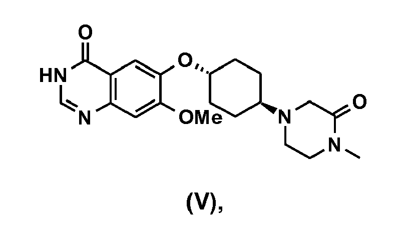 while the steps take place successively in the order specified.

2. Process according to claim 1 for the stereoselective preparation of compounds of general formula **(I), characterised in that** the process consists of process steps **(1a)** to **(1d).**

3. Process according to claim 1 for the stereoselective preparation of compounds of general formula **(I), characterised in that** it consists of process steps **(2a), (1c)** and **(1d).**

4. Process according to claim 1 for the stereoselective preparation of compounds of general formula **(IV), characterised in that** the process consists of process step **(1a).**

5. Process according to claim 1 for the stereoselective preparation of compounds of general formula **(V), characterised in that** the process consists of process step **(1 b).**

6. Process according to claim 1 for the stereoselective preparation of compounds of general formula **(VI), characterised in that** the process consists of process step **(1c).**

7. Process according to claim 1 for the stereoselective preparation of compounds of general formula **(I), characterised in that** the process consists of process step **(1d).**

8. Process according to claim 1 for the stereoselective preparation of compounds of general formula **(IV), characterised in that** the process consists of process step **(2a).**

9. Compound of formula **(II)**

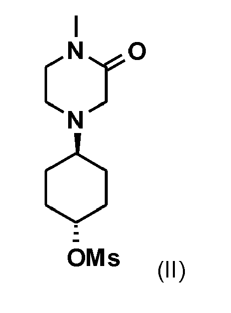

10. Compound of formula **(VIII)**

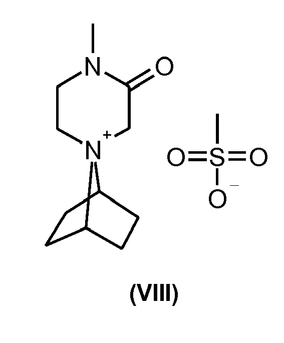

## Revendications

1. Procédé de production stéréosélective de composés de formule générale (I) 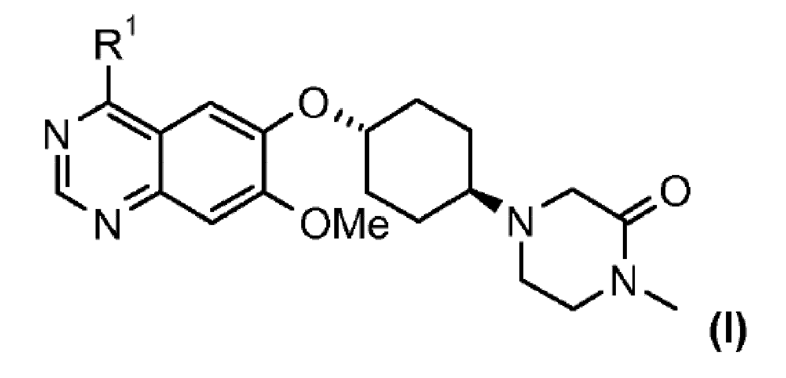 Eventuellement sous la forme de leur tautomères, ainsi qu'éventuellement de leurs sels d'addition d'acide pharmacologiquement acceptable,
où
R¹ représente un radical choisi parmi le groupe consistant en le 3-chloro-2-fluoro-phénylamino, le 3-chloro-4-fluoro-phénylamino, le 2-fluoro-3-méthyl-phénylamino-, le 2,5-difluoro-3-méthyl-phénylamino-, le 3-chloro-2-méthyl-phénylamino et le 2-fluoro-5-méthyl-phénylamino-,
**caractérisé en ce que** le procédé comprend les étapes de réaction (1a) à (1d),
(1a) représentant la mise en réaction d'un composé de formule (II) 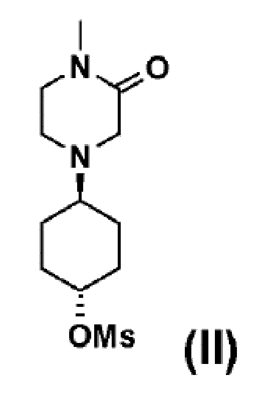 avec un composé de formule (III) 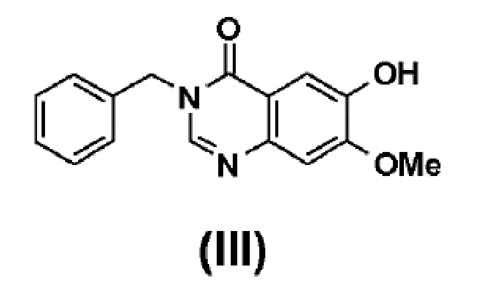 pour former un composé de formule (IV) 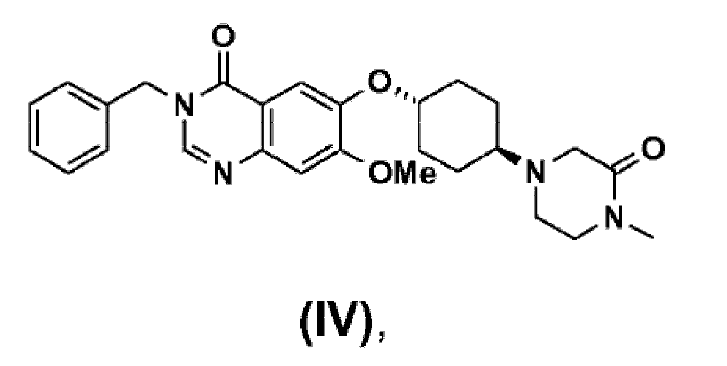
(1b) représentant la scission du radical benzyle du composé de formule (IV) en présence d'un catalyseur pour former un composé de formule (V) 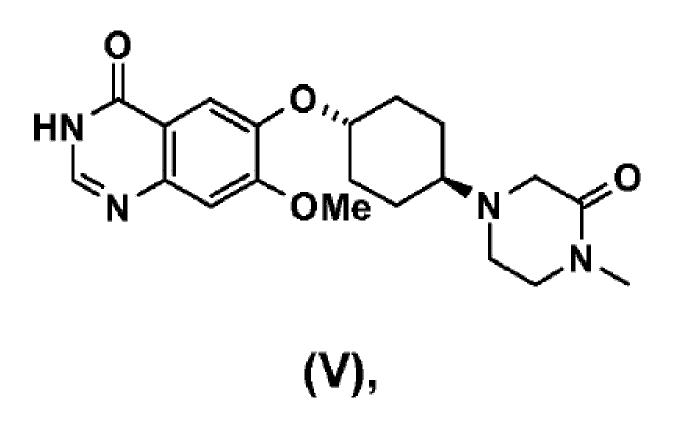
(1c) représentant la mise en réaction du composé de formule (V) avec un agent de chloration pour former le chlorhydrate d'un composé de formule (VI) 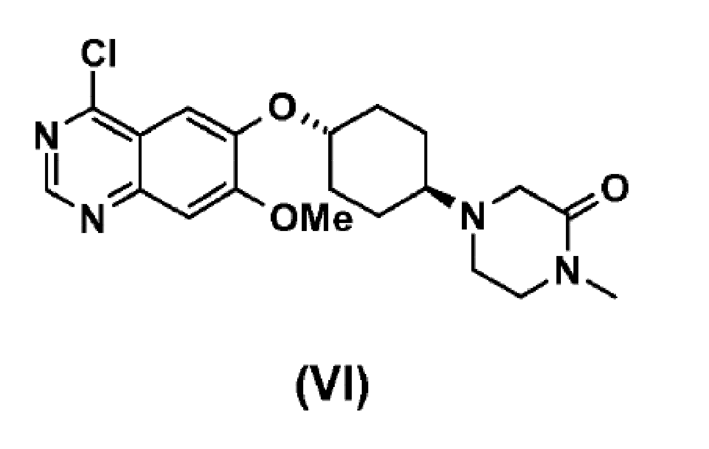 et
(1d) représentant la mise en réaction du composé de formule (VI) avec un des composés (i) à (vi) pour former un composé de formule (I)
(i) représentant de la 3-chloro-2-fluoro-aniline,
(ii) représentant de la 3-chloro-4-fluoro-aniline,
(ii) représentant de la 2-fluoro-3-méthyl-aniline,
(iii) représentant de la 2,5-difluoro-3-méthyl-aniline,
(iv) représentant de la 3-chloro-2-méthyl-aniline, et
(vi) représentant de la 2-fluoro-5-méthyl-aniline,
les étapes (1a) à (1d) étant réalisées les unes après les autres dans l'ordre évoqué
ou
**caractérisé en ce que** le procédé comprend les étapes de réaction (2a), (1c) et (1d),
(2a) représentant la mise en réaction d'un composé de formule (II) 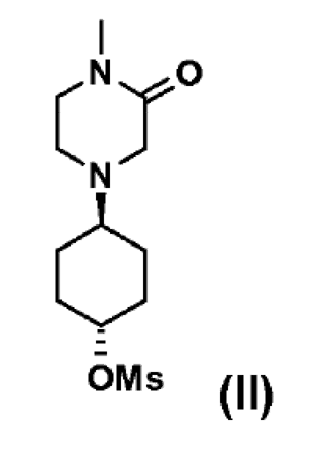 avec un composé de formule (VII) 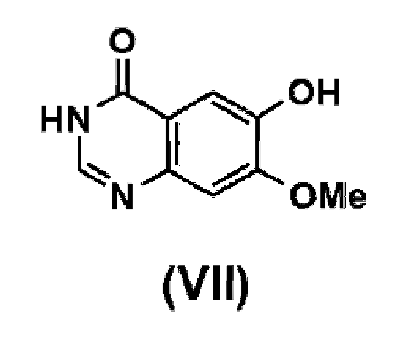 pour former un composé de formule (V) 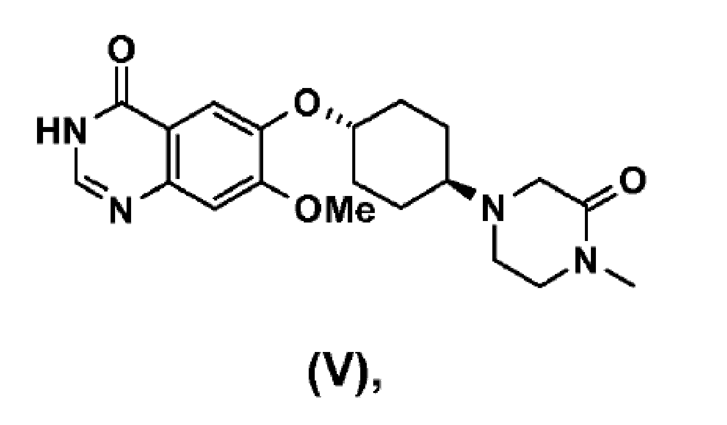 les étapes étant réalisées les unes après les autres selon l'ordre mentionné.

2. Procédé selon la revendication 1 de production stéréosélective de composés de formule générale (I), **caractérisé en ce que** le procédé consiste en les étapes de procédé (1a) à (1d).

3. Procédé selon la revendication 1 de production stéréosélective de composés de formule générale (I), **caractérisé en ce que** le procédé consiste en les étapes de procédé (2a), (1c), et (1d).

4. Procédé selon la revendication 1 de production stéréosélective de composés de formule générale (IV), **caractérisé en ce que** le procédé consiste en l'étape de procédé (1a).

5. Procédé selon la revendication 1 de production stéréosélective de composés de formule générale (V), **caractérisé en ce que** le procédé consiste en l'étape de procédé (1b).

6. Procédé selon la revendication 1 de production stéréosélective de composés de formule générale (VI), **caractérisé en ce que** le procédé consiste en l'étape de procédé (1c).

7. Procédé selon la revendication 1 de production stéréosélective de composés de formule générale (I), **caractérisé en ce que** le procédé consiste en l'étape de procédé (1d).

8. Procédé selon la revendication 1 de production stéréosélective de composés de formule générale (IV), **caractérisé en ce que** le procédé consiste en l'étape de procédé (2a).

9. Composé de formule (II)

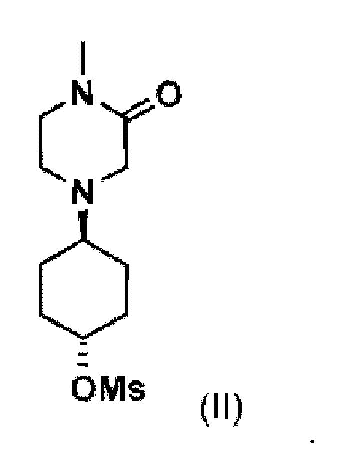

10. Composé de formule (VIII)
